# EUROPEAN PATENT SPECIFICATION

(11) **EP 1 818 059 B1**
(45) Date of publication and mention of the grant of the patent: **03.12.2008**
(21) Application number: 05819681.7
(22) Date of filing: 22.12.2005
(51) Int. Cl.: A61K 31/551, A61K 33/42, A61P 27/06

(54) **PREVENTIVE OR THERAPEUTIC AGENT FOR GLAUCOMA**
PRÄVENTIVER ODER THERAPEUTISCHER WIRKSTOFF GEGEN GLAUKOM
AGENT DE PREVENTION OU DE TRAITEMENT DU GLAUCOME

(30) Priority: 23.12.2004 US 638118 P
(43) Date of publication of application: 15.08.2007
(73) Proprietor: Kowa Company, Ltd., Naka-ku Nagoya-shi, Aichi-ken 460-8625 (JP)
(72) Inventor: KANEBAKO, Makoto, Fuji-Shi, Shizuoka 4178650 (JP); TAKAHASHI, Masatoshi, Higashiyamato-shi Tokyo 207-0033 (JP); MIZUNO, Ken, Tokyo 189-0022 (JP); SUGIMOTO, Shin, Fuji-shi, Shizuoka 4178650 (JP)
(74) Representative: Hartz, Nikolai
(86) International application number: PCT/JP2005/023570
(87) International publication number: WO 2006/068208

(56) References cited:
- WO-A-97/23222
- JP-A- 10 310 576
- JP-A- 11 349 482
- JP-A- 2001 509 780
- SASAKI YASUHARU ET AL: "The novel and specific Rho-kinase inhibitor (S)-(+)-2-methyl-1-((4-me thyl-5-isoquinoline)sulfonyl)-homopiperazi ne as a probing molecule for Rho-kinase-involved pathway" PHARMACOLOGY AND THERAPEUTICS, vol. 93, no. 2-3, February 2002 (2002-02), pages 225-232, XP002458450 ISSN: 0163-7258
- HONJO M. ET AL.: 'Effects of Protein Kinase Inhibitor, HA1077, on Intraocular Pressure and Outflow Facility in Rabbit Eyes' ARCH. OPHTHALMOL. vol. 119, no. 8, 2001, pages 1171 - 1178, XP002999418

## Description

### FIELD OF THE INVENTION

This invention relates to a preventing or treating agent for glaucoma comprising (S)- (-) -1- (4-fluoro-5-isoquinolinesulfonyl) -2-methyl homopiperazine or its salt and phosphoric acid or its salt.

### BACKGROUND OF THE INVENTION

In the eye, aqueous humor continuously circulates to maintain intraocular pressure at a constant level. However, when flow rate of the aqueous humor at the trabecular meshwork which is the outlet of the aqueous humor reduces, or when corner angle narrows to block the flow of the aqueous humor, intraocular pressure will be raised to press the optic nerve, and this results in the onset of glaucoma associated with abnormality in visual field, or ocular hypertension which might not be associated with such abnormal visual field but which is likely to develop into glaucoma in a long term. In treating such glaucoma or ocular hypertension, the increased intraocular pressure should be reduced, and in the clinical practice, sympathetic stimulants such as epinephrine, parasympathetic stimulants such as pilocarpine hydrochloride, β blockers such as timolol, prostaglandin analogs such as isopropyl unoprostone, carbonic anhydrase inhibitors such as dorzolamide have been used. None of these drugs, however, have proved to be sufficient in their action of reducing the intraocular pressure.

Recently, other treatments have also been reported including the treatment by a combination of a β blocker, a prostaglandin analog, and a carbonic anhydrase inhibitor in order to enhance the intraocular pressure-reducing action (Non-Patent Document), and the treatment by a combination of a β blocker and alginic acid to sustain the intraocular pressure-reducing action (Patent document 1) . Another recent report discloses that a compound having Rho kinase inhibitory action has excellent preventing and treating effects for glaucoma as well as strong intraocular pressure-reducing action (Patent Document 2).

Many of the glaucoma patients suffer from the type of glaucoma (normal tension glaucoma) not associated with the increase of intraocular pressure. In the case of such patients, reduction of the intraocular pressure from the normal level is required. Reducing the intraocular pressure from the normal level, however, is harder than reducing the intraocular pressure at an elevated level, and this imposed a certain limitation on the treatment of the normal tension glaucoma by the conventional drug or combination of such conventional drugs. Accordingly, there has been a demand for a drug with an enhanced intraocular pressure-reduction action.
Non-Patent Document: Clinical Practice in Opthalmology 4(5): 2-6, 2001, in Japanese
Patent Document 1: JP-A-2002-511430
Patent Document 2: WO 00/9162
The document WO 97/23222 discloses the reduction of intraocular pressure in animal studies following the topical administration of a composition comprising the Rho-kinase inhibitor fasudil hydrochloride and a phosphate buffer.

### DISCLOSURE OF THE INVENTION

### PROBLEMS TO BE SOLVED BY THE INVENTION

An object of the present invention is to provide a preventing or treating agent for glaucoma which has strong action of reducing the intraocular pressure, namely an action sufficiently strong to reduce the intraocular pressure from the normal level.

### MEANS FOR SOLVING THE PROBLEMS

The inventors of the present invention have made an extensive study to solve the problems as described above, and found that a strong intraocular pressure-reducing action can be realized when (S)-(-)-1-(4-fluoro-5-isoquinolinesulfonyl)-2-methyl homopiperazine or its salt which is a known prophylactic or therapeutic agent for asthma and known to have antagonistic action for substance P, antagonistic action for leukotriene D4, and inhibitory action on Rho kinase is used in combination with phosphoric acid or its salt. The present invention has been completed based on such findings.

Accordingly, this invention relates to a preventing or treating agent for glaucoma comprising (S)-(-)-1-(4-fluoro-5-isoquinolinesulfonyl)-2-methyl homopiperazine or its salt and phosphoric acid or its salt.

This invention also relates to a preventing or treating agent for ocular hypertension comprising (S)-(-)-1-(4-fluoro-5-isoquinolinesulfonyl)-2-methyl homopiperazine or its salt and phosphoric acid or its salt.

This invention also relates to eye drops comprising (S)-(-)-1-(4-fluoro-5-isoquinolinesulfonyl)-2-methyl homopiperazine or its salt and phosphoric acid or its salt.

This invention also relates to use of (S)-(-)-1-(4-fluoro-5-isoquinolinesulfonyl)-2-methyl homopiperazine or its salt and phosphoric acid or its salt for producing a preventing or treating agent for glaucoma.

This invention also relates to use of (S)-(-)-1-(4-fluoro-5-isoquinolinesulfonyl)-2-methyl homopiperazine or its salt and phosphoric acid or its salt for producing a prevent ing or treating agent for ocular hypertension.

This invention also relates to use of (S)-(-)-1-(4-fluoro-5-isoquinolinesulfonyl)-2-methyl homopiperazine or its salt and phosphoric acid or its salt for producing eye drops.

Further, a method for preventing or treating glaucoma wherein (S)-(-)-1-(4-fluoro-5-isoquinolinesulfonyl)-2-methyl homopiperazine or its salt and phosphoric acid or its salt is administered is described.

Further, a method for preventing or treating ocular hypertension wherein (S) - (-) -1- (4-fluoro-5-isoquinolinesulfonyl)-2-methyl homopiperazine or its salt and phosphoric acid or its salt is administered is described

### EFFECT OF THE INVENTION

The preventing or treating agent for glaucoma of the present invention have strong action of reducing the intraocular pressure, and the action is so strong that the intraocular pressure can be sufficiently reduced even from the normal level. Accordingly, use of such agent realizes significant preventing or treating effects for glaucoma or ocular hypertension in a reduced number of administrations.

### BRIEF DESCRIPTION OF THE DRAWINGS

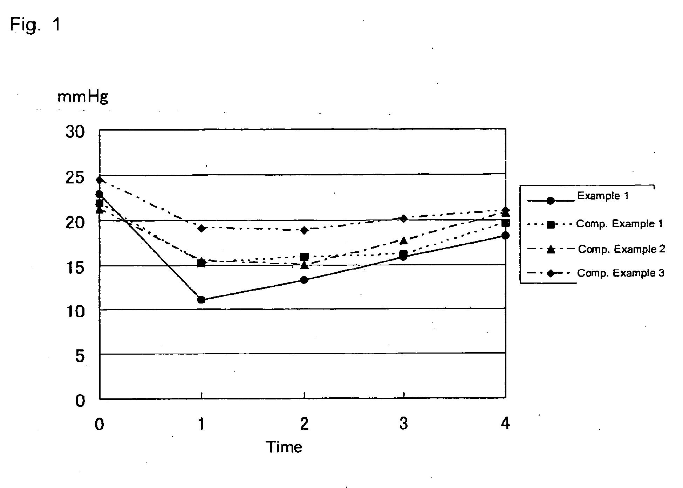

FIG. 1 is a view showing the action of reducing the intraocular pressure in the present preparation.

### THE BEST MODE FOR CARRYING OUT THE INVENTION

The preventing or treating agent for glaucoma, the preventing or treating agent for ocular hypertension, and eye drops of the present invention comprise (S) -(-) -1- (4-fluoro-5-isoquinolinesulfonyl) -2-methyl homopiperazine or its salt and phosphoric acid or its salt.

(S)-(-)-1-(4-Fluoro-5-isoquinolinesulfonyl)-2-methyl homopiperazine is a known compound (JP-A-11-349482) having antagonistic action for substance P, antagonistic action for leukotriene D4, and inhibitory action on Rho kinase, and this compound can be produced by the method described, for example, in WO 99/20620.

Examples of the salt of (S)-(-)-1-(4-fluoro-5-isoquinolinesulfonyl)-2-methyl homopiperazine include inorganic acid salts such as hydrochloride, sulfate, nitrate, hydrofluoride, and hydrobromide, and organic acid salts such as acetate, tartarate, lactate, citrate, fumarate, malate, succinate, methanesulfonate, ethanesulfonate, benzenesulfonate, toluenesulfonate, naphthalenesulfonate, and camphorsulfonate. The preferred is hydrochloride.

The (S)-(-)-1-(4-fluoro-5-isoquinolinesulfonyl)-2-methyl homopiperazine and its salt may be present in unsolvated form, and also, as a hydrate or a solvate, and all crystal forms, hydrates, and solvates.

Examples of the phosphoric acid or its salt include phosphoric acid, disodium hydrogenphosphate, dipotassium hydrogenphosphate, sodium dihydrogenphosphate, and potassium dihydrogenphosphate, and the most preferred is sodium dihydrogenphosphate.

In the preventing or treating agent for glaucoma, the preventing or treating agent for ocular hypertension, and the eye drops of the present invention, (S)-(-)-1-(4-fluoro-5-isoquinolinesulfonyl)-2-methyl homopiperazine or its salt is preferably incorporated at a content of 0.1 to 5 % by weight, more preferably at 0.1 to 3 % by weight, and most preferably at 0 .1 to 2 % by weight in relation to total weight of the drug composition. The phosphoric acid or its salt is preferably incorporated at a content of 0.01 to 5 % by weight, more preferably at 0. 1 to 3 % by weight, and most preferably at 0.1 to 1 % by weight in relation to total weight of the drug composition.

When (S)-(-)-1-(4-fluoro-5-isoquinolinesulfonyl)-2-methyl homopiperazine or its salt is used in combination with phosphoric acid or its salt, it exhibits excellent action of reducing the intraocular pressure even from the normal intraocular pressure level as will be demonstrated later in the Test Examples. Therefore, the drug containing such combination of reagents is useful as a preventing or treating agent for glaucoma or as a preventing or treating agent for ocular hypertension. The glaucoma postulated include primary open angle glaucoma, normal tension glaucoma, glaucoma producing excessive aqueous humor, ocular hypertension, acute angle closure glaucoma, chronic angle closure glaucoma, plateau iris syndrome, mixed glaucoma, steroid glaucoma, capsular glaucoma, pigmentary glaucoma, amyloid glaucoma, and neovascular glaucoma, and malignant glaucoma.

The preventing or treating agent for glaucoma or ocular hypertension of the present invention is preferably used as an ophthalmic preparation, and in particular, in the form of eye drops. Such eye drops may be any of aqueous eye drops, non-aqueous eye drops, suspension eye drops, emulsion eye drops, ophthalmic ointment.

The eye drops of the present invention may have any osmotic pressure and pH. The pH, however, is typically in the range of 5 to 9, preferably 5 to 8, and most preferably 5 to 7, and the osmotic pressure is typically 200 to 700 mOsm/Kg, and preferably 200 to 600 mOsm/Kg. Ratio of the osmotic pressure in relation to that of the physiological saline is preferably in the range of 0.6 to 3, and most preferably 0.6 to 2. When the pH and the osmotic pressure is within such range, the resulting preparation will be less stimulative to the eye upon its application.

If necessary, the eye drops of the present invention may also contain an isotonic agent, a chelating agent, a stabilizer, a pH adjusting agent, an antiseptic, an antioxidant, a solubilizer, a thickener, and other effective components in addition to the essential components, that is, the (S)-(-)-1-(4-fluoro-5-isoquinolinesulfonyl)-2-methyl homopiperazine or its salt and the phosphoric acid or its salt .

Exemplary isotonic agents include saccharides such as glucose, trehalose, lactose, fructose, mannitol, xylitol, and sorbitol; polyhydric alcohols such as glycerin, polyethyleneglycol, and propylene glycol; and inorganic salts such as sodium chloride, potassium chloride, and calcium chloride; and such isotonic agent is preferably incorporated at a content of 0 to 5 % by weight in relation to the total weight of the composition.

Exemplary chelating agents include edetates such as disodium edetate, calcium disodium edetate, trisodium edetate, tetrasodium edetate, and calcium edetate; ethylenediaminetetraacetic acid, nitrilotriacetic acid or its salt, sodium hexametaphosphate, and citric acid; and such isotonic agent is preferably incorporated at a content of 0 to 0 .2 % by weight in relation to the total weight of the composition.

Exemplary stabilizers include sodium hydrogen sulfite; and such stabilizer is preferably incorporated at 0 to 1% by weight in relation to the total weight of the composition.

Exemplary pH adjusting agents include acids such as hydrochloric acid, carbonic acid, acetic acid, and citric acid; alkaline metal carbonate or hydrogencarbonate such as sodium hydroxide and potassium hydroxide; alkaline metal acetates such as sodium acetate; alkaline metal citrates such as sodium citrate; and bases such as trometamol; and such pH adjusting agent is preferably incorporated at 0 to 20% by weight in relation to the total weight of the composition.

Exemplary antiseptics include sorbic acid, potassium sorbate, methyl paraoxybenzoate, ethyl paraoxybenzoate, propyl paraoxybenzoate, butyl paraoxybenzoate and other paraoxybenzoates; chlorhexidine gluconate, benzalkonium chloride, benzethonium chloride, cetylpyridinium chloride and other quaternary ammonium salts; alkylpolyaminoethylglycine, chlorobutanol, polyquad, polyhexamethylene biguanide, and chlorhexidine; and such antiseptic is preferably incorporated at 0 to 0.2 % by weight in relation to the total weight of the composition.

Exemplary antioxidants include sodium hydrogen sulfite, dry sodium hydrogen sulfite, sodium pyrosulfite, concentrated mixed tocopherol; and such antioxidant is preferably incorporated at 0 to 0.4% by weight in relation to the total weight of the composition.

Exemplary solubilizers include sodium benzoate, glycerin, D-sorbitol, glucose, propylene glycol, hydroxypropyl methylcellulose, polyvinyl pyrrolidone, macrogol, and D-mannitol, and such solubilizer is preferably incorporated at 0 to 3% by weight in relation to the total weight of the composition.

Exemplary thickeners include polyethyleneglycol, methyl cellulose, ethyl cellulose, carmellose sodium, xanthan gum, chondroitin sulfate sodium, hydroxyethyl cellulose, hydroxypropyl cellulose, hydroxypropyl methylcellulose, polyvinyl pyrrolidone, and polyvinyl alcohol, and such solubilizer is preferably incorporated at 0 to 70% by weight in relation to the total weight of the composition.

Examples of other effective components include non-receptor selective sympathetic stimulants, α-2 receptor selective sympathetic stimulants, and other sympathetic stimulants, non-selective β receptor blocker, β-1 receptor blocker, α-β receptor blocker, α-1 receptor blocker and other sympathetic stimulants, parasympathetic stimulants, prostaglandin analogs, carbonic anhydrase inhibitors, hyperosmotic agents, muscarinergic agents, cholinesterase inhibitors, and glutamate antagonists.

The eye drops of the present invention may be prepared by dissolving or suspending the desired components as described above in an aqueous solvent such as sterilized purified water, or physiological saline, or in a non-aqueous solvent such as cottonseed oil, soybean oil, sesameoil, orpeanutoil, adjusting the osmotic pressure to a predetermined range, and sterilizing the solution or the suspension for example, by filter sterilization. When an ophthalmic ointment is prepared, the composition may further include an ointment base in addition to the components as described above. Exemplary preferable ointment bases include oil bases such as vaseline, liquid paraffin, and polyethylene; emulsion bases having oil phase and water phase emulsified by a surfactant, and water-soluble bases such as hydroxypropyl methylcellulose, carboxymethylcellulose, and polyethyleneglycol.

In administering the preventing or treating agent for glaucoma or the prophylactic or therapeutic agent for ocular hypertension of the present invention, the dose may be adjusted by considering age, body weight, and other conditions of the patient, administration route, nature and seriousness of the disease. The dose, however, is generally 0.05 to 10 mg, and preferably 0.1 to 5 mg per day per adult in terms of the effective ingredient, and this dose may be administered in a single dose or in 2 to several divided doses per day. In the case of the liquid eye drops, 1 to several drops may be administered at one time.

### EXAMPLES

Next, the present invention is described in further detail by referring to Examples and Comparative Examples.

### Example 1

To 1.1 g of (S) - (-) -1- (4-fluoro-5-isoquinolinesulfonyl) -2-methyl homopiperazine hydrochloride, 0.8 g of anhydrous sodium dihydrogenphosphate (manufactured by Taihei Chemical Industrial Co. , Ltd.), and 0. 5 g of potassium chloride was added 50 g of purified water, and the mixture was stirred for complete dissolution. To this solution was added solution of sodium hydroxide in purified water to adjust pH to 6.7. Purified water was further added to a total volume of 100 g. Osmotic pressure was 348 mOsm/Kg.

### Example 2

Eye drops were prepared by repeating the procedure of Example 1 by using 0 . 6 g of anhydrous sodium dihydrogenphosphate. Osmotic pressure was 332 mOsm/Kg.

### Example 3

Eye drops were prepared by repeating the procedure of Example 1 by using 0.4 g of anhydrous sodium dihydrogenphosphate. Osmotic pressure was 315 mOsm/Kg.

### Example 4

Eye drops were prepared by repeating the procedure of Example 1 by using 0.2 g of anhydrous sodium dihydrogenphosphate. Osmotic pressure was 301 mOsm/Kg.

### Example 5

To 0.55 g of (S)-(-)-1-(4-fluoro-5-isoquinolinesulfonyl)-2-methyl homopiperazine hydrochloride, 0.4 g of anhydrous sodium dihydrogenphosphate (manufactured by Taihei Chemical Industrial Co. , Ltd.), and 0.96 g of potassium chloride was added 50 g of purified water, and the mixture was stirred for complete dissolution. To this solution was added solution of sodium hydroxide in purified water to adjust pH to 6 . 7. Purified water was further added to a total volume of 100 g. Osmotic pressure was 313 mOsm/Kg.

### Example 6

To 0.275 g of (S) - (-) -1- (4-fluoro-5-isoquinolinesulfonyl)-2-methyl homopiperazine hydrochloride, 0.4 g of anhydrous sodium dihydrogenphosphate (manufactured by Taihei Chemical Industrial Co. , Ltd.), and 0.99 g of potassium chloride was added 50 g of purified water, and the mixture was stirred for complete dissolution. To this solution was added solution of sodium hydroxide in purified water to adjust pH to 6.7. Purified water was further added to a total volume of 100 g. Osmotic pressure was 315 mOsm/Kg.

### Comparative Example 1

To 1.1 g of (S)-(-)-1-(4-fluoro-5-isoquinolinesulfonyl)-2-methyl homopiperazine hydrochloride and 1 g of potassium chloride was added 50 g of purified water, and the mixture was stirred for complete dissolution. To this solution was added solution of sodium hydroxide in purified water to adjust pH to 6 . 7 . Purified water was further added to a total volume of 100 g. Osmotic pressure was 344 mOsm/Kg.

### Comparative Example 2

To 1.1 g of (S)-(-)-1-(4-fluoro-5-isoquinolinesulfonyl)-2-methyl homopiperazine hydrochloride, 0.9 g of potassium chloride, 0.1 g of alginic acid (Duckacid A manufactured by Kibun Food Chemifa Co., Ltd.) was added 50 g of purified water, and the mixture was stirred for complete dissolution. To this solution was added solution of sodium hydroxide in purified water to adjust pH to 6.7. Purified water was further added to a total volume of 100 g. Osmotic pressure was 340 mOsm/Kg.

### Comparative Example 3

*0.* 5% timolol maleate eye drops (product name, Timoptol (R) 0.5%; manufactured by Banyu Pharmaceutical Co., Ltd. and sold by Santen Pharmaceutical Co., Ltd.; lot number, 9AC19P) were used. The pH was 6.9, and osmotic pressure was 270 mOsm/Kg.

### Test Example 1

Male Japanese white rabbits with normal intraocular pressure (2.0 to 2. 5 Kg) were placed in a rabbit fixer with no anesthetization, and a drop of Benoxil (Santen Pharmaceutical Co., Ltd.) was instilled in both eyes of the rabbit for local anesthesia of the eyes. Intraocular pressure of both eyes was then measured with applanation pneumatonometer (Alcon Applanation Pneumatonograph (TM), Alcon Japan), and the value measured was used as 0-hour value. After measuring the 0-hour value, 50 µl of the eye drops of Examples 1 to 4 and Comparative Examples 1 to 3 were administered to the left eye, and intraocular pressure of this eye was measured at 1 hour, 2 hours, 3 hours, and 4 hours after the instillation (n = 4 to 6). The results are shown in Table 1 and FIG. 1 (The results of Example 1 and Comparative Examples 1 to 3).

**Table 1**

| | | | | | | | |
|---|---|---|---|---|---|---|---|
| (mmHg) | | | | | | | |

| | Ex. 1 | Ex. 2 | Ex. 3 | Ex. 4 | Comp. Ex. 1 | Comp. Ex. 2 | Comp. Ex. 3 |
|---|---|---|---|---|---|---|---|
| The value after 0 hour | 22.9 | 23.8 | 26.6 | 21.7 | 21.9 | 21.2 | 24.4 |
| The value after 1 hour | 11.1 | 11.9 | 12.2 | 10.7 | 15.2 | 15.4 | 19.1 |
| The value after 2 hours | 13.3 | 12.3 | 14.9 | 14.3 | 15.9 | 14.9 | 18.8 |
| The value after 3 hours | 15.8 | 15.2 | 19.4 | 16.8 | 16.1 | 17.6 | 20.1 |
| The value after 4 hours | 18.2 | 16.7 | 20.8 | 22.2 | 19.6 | 20.7 | 21.0 |

As demonstrated in Table 1 and FIG. 1, the preparations of the present invention (Examples 1 to 4) exhibit superior action of reducing the intraocular pressure even fromnormal intraocular pressure compared to the preparation containing no phosphoric acid (Comparative Example 1), the preparation containing alginic acid instead of the phosphoric acid (Comparative Example 2), and the widely used commercially available timolol eye drops (Comparative Example 3).
The eye drops of Examples 5 and 6 also exhibited equally excellent action of reducing the intraocular pressure.

## Claims

1. A preventing or treating agent for glaucoma or for ocular hypertension, said agent comprising (S)-(-)-1-(4-fluoro-5-isoquinolinesulfonyl)-2-methyl homopiperazine or its salt and phosphoric acid or its salt.

2. Eye drops comprising (S)-(-)-1-(4-fluoro-5-isoquinolinesulfonyl)-2-methyl homopiperazine or its salt and phosphoric acid or its salt.

3. Use of (S)-(-)-1-(4-fluoro-5-isoquinolinesulfonyl)-2-methyl homopiperazine or its salt and phosphoric acid or its salt for producing a prophylactic or therapeutic agent for glaucoma.

4. Use of (S)-(-)-1-(4-fluoro-5-isoquinolinesulfonyl)-2-methyl homopiperazine or its salt and phosphoric acid or its salt for producing a preventing or treating agent for ocular hypertension.

5. Use of (S)-(-)-1-(4-fluoro-5-isoquinolinesulfonyl)-2-methyl homopiperazine or its salt and phosphoric acid or its salt for producing eye drops.

## Patentansprüche

1. Mittel zur Prävention oder Behandlung von Glaukom oder Augenhochdruck, das (S)-(-)-1-(4-Fluor-5-isochinolinsulfonyl)-2-methylhomopiperazin oder sein Salz und Phosphorsäure oder ihr Salz aufweist.

2. Augentropfen, die (S)-(-)-1-(4-Fluor-5-isochinolinsulfonyl)-2-methylhomopiperazin oder sein Salz und Phosphorsäure oder ihr Salz aufweisen.

3. Verwendung von (S)-(-)-1-(4-Fluor-5-isochinolinsulfonyl)-2-methylhomopiperazin oder seines Salzes und Phosphorsäure oder ihres Salzes zur Herstellung eines prophylaktischen oder therapeutischen Mittels für Glaukom.

4. Verwendung von (S)-(-)-1-(4-Fluor-5-isochinolinsulfonyl)-2-methylhomopiperazin oder seines Salzes und Phosphorsäure oder ihres Salzes zur Herstellung eines Mittels zur Prävention oder Behandlung von Augenhochdruck.

5. Verwendung von (S)-(-)-1-(4-Fluor-5-isochinolinsulfonyl)-2-methylhomopiperazin oder seines Salzes und Phosphorsäure oder ihres Salzes zur Herstellung von Augentropfen.

## Revendications

1. Agent de prévention ou de traitement du glaucome ou de l'hypertension oculaire, ledit agent comprenant de la (S)-(-)-1-(4-fluoro-5-isoquinolinesulfonyl)-2-méthylhomopipérazine ou son sel et de l'acide phosphorique ou son sel.

2. Collyre comprenant de la (S)-(-)-1-(4-fluoro-5-isoquinolinesulfonyl)-2-méthylhomopipérazine ou son sel et de l'acide phosphorique ou son sel.

3. Utilisation de la (S) - (-) -1- (4-fluoro-5-isoquinolinesulfonyl)-2-méthylhomopipérazine ou de son sel et de l'acide phosphorique ou de son sel pour la production d'un agent prophylactique ou thérapeutique pour le glaucome.

4. Utilisation de la (S)-(-)-1-(4-fluoro-5-isoquinolinesulfonyl)-2-méthylhomopipérazine ou de son sel et de l'acide phosphorique ou de son sel pour la production d'un agent de prévention ou de traitement de l'hypertension oculaire.

5. Utilisation de la (S)-(-)-1-(4-fluoro-5-isoquinolinesulfonyl)-2-méthylhomopipérazine ou de son sel et de l'acide phosphorique ou de son sel pour la production d'un collyre.
